(19) Europäisches Patentamt
European Patent Office
Office européen des brevets

(11) Publication number: 0 038 614 A2

# (12) EUROPEAN PATENT APPLICATION

(21) Application number: 81200688.0

(22) Date of filing: 13.08.79

(51) Int. Cl.³: C 07 C 177/00
A 61 K 31/557

(30) Priority: 14.08.78 US 933329

(43) Date of publication of application: 28.10.81 Bulletin 81/43

(84) Designated Contracting States: BE CH DE FR GB IT NL

(60) Publication number of the earlier application in accordance with Art. 76 EPC: 0 009 869

(71) Applicant: THE UPJOHN COMPANY
301 Henrietta Street
Kalamazoo, Michigan 49001(US)

(72) Inventor: Sih, John Charles
419 Ellendale
Kalamazoo Michigan(US)

(74) Representative: Perry, Robert Edward et al,
GILL JENNINGS & EVERY 53-64 Chancery Lane
London WC2A 1HN(GB)

## (54) Esters of 5- and 6-oxo-prostaglandins.

(57) ($C_{1-4}$ alkyl)carbonylphenyl esters of prostaglandin compounds, for example the 4-acetylphenyl esters of 5-oxo-$PGF_{1\alpha}$ and 6-oxo-$PGF_{1\alpha}$ compounds. The esters can have pharmacological activity.

EP 0 038 614 A2

# ESTERS OF 5- AND 6-OXO-PROSTAGLANDINS

This invention relates to esters of 6-oxo-prostaglandins and, in particular, to acylphenyl esters of such compounds.

Many prostaglandins are known. A background discussion of such compounds is given by Bergstrom et al., Pharmacol. Rev.20, 1 (1968).

6-Oxo-$PGF_{1\alpha}$ is described by Pace-Asciak, J. Am. Chem. Soc. 98, 2348 (1976). Acetylphenyl esters of certain prostaglandins are described in U.S. Patent Specifications Nos. 3,890,372 and 3,894,062.

The nomenclature of prostaglandins is described by N.A. Nelson, J. Medic. Chem. 17, 911 (1974). With respect to "R" and "S" usage, as for substitution at C-6 and C-15 (having regard to the numbering of the carbon atoms in prostanoic acid), see R.S. Cahn, J. Chem. Ed. 41, 116 (1964).

The novel compounds of this invention have the formula

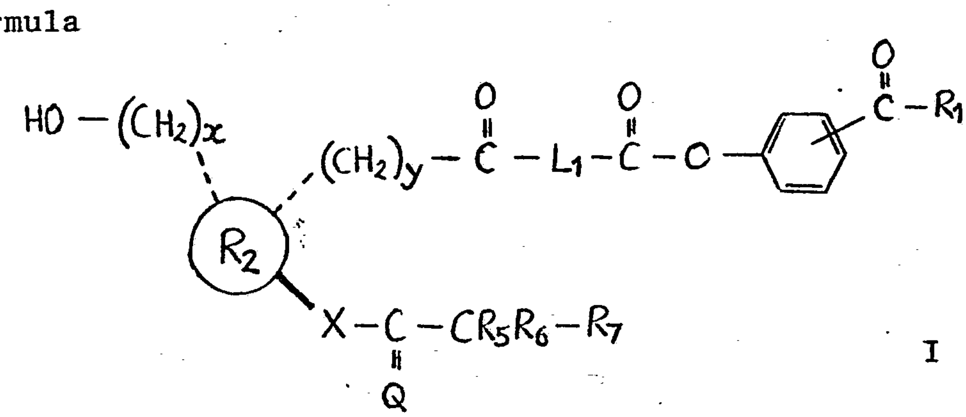

I

wherein x is zero or one, y is one or 2, and x plus y is one or 2;

$L_1$ is $-(CH_2)_n-$, $-(CH_2)_p-CF_2-$ or $-(CH_2)_v-CH=CH-$ and n is an integer of from one to 5, p is 2, 3 or 4 and v is one, 2 or 3;

Q is oxo, α-H:β-H, α-OH:β-$R_4$ or α-$R_4$:β-OH and $R_4$ is hydrogen or $C_{1-4}$ alkyl;

$R_1$ is $C_{1-4}$ alkyl, provided that $-COR_1$ is in the 4-position on the benzene ring when $R_1$ is _tert_-butyl;

($R_2$) is

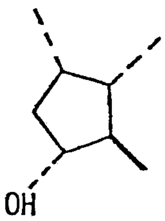
,
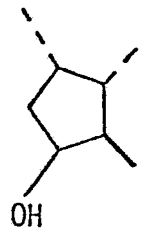
,
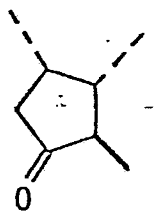
,
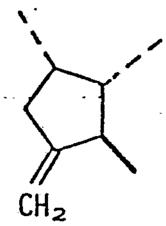
,
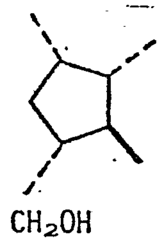
,
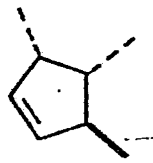
or
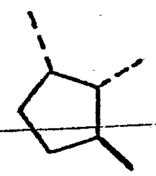

$R_7$ is $C_{2-10}$ alkyl (with a straight chain no longer than 6 carbon atoms) or optionally ring-substituted phenoxy, phenyl or phenyl($C_{1-9}$ alkyl) with no more than 6 carbon atoms in the chain between $CR_5R_6$ and the benzene ring, in which there are up to 3 ring substituents independently selected from fluorine, chlorine, trifluoromethyl, $C_{1-4}$ alkyl and $C_{1-4}$ alkoxy, provided that no more than two substituents are other than alkyl;

$R_5$ and $R_6$ are independently selected from hydrogen, fluorine and $C_{1-4}$ alkyl, with the provisos that $CR_5R_6$ is not CFAlkyl and that neither $R_5$ nor $R_6$ is fluorine when $R_7$ is optionally ring-substituted phenoxy; and

X is _trans_-CH=CH-, _cis_-CH=CH-, -C≡C- or $-CH_2CH_2-$.

The 9- or 9'-hydroxyl group and the 6(or 5)-oxo group in the compounds of this invention can undergo intramolecular reaction to form a corresponding prostacyclin compound of the type described and claimed

in European Patent Application No. 79301635.3 (Serial No. 9869). At least in solution, the prostacyclin and prostaglandin compounds are in equilibrium. The analogous prostacyclin compounds have the formula

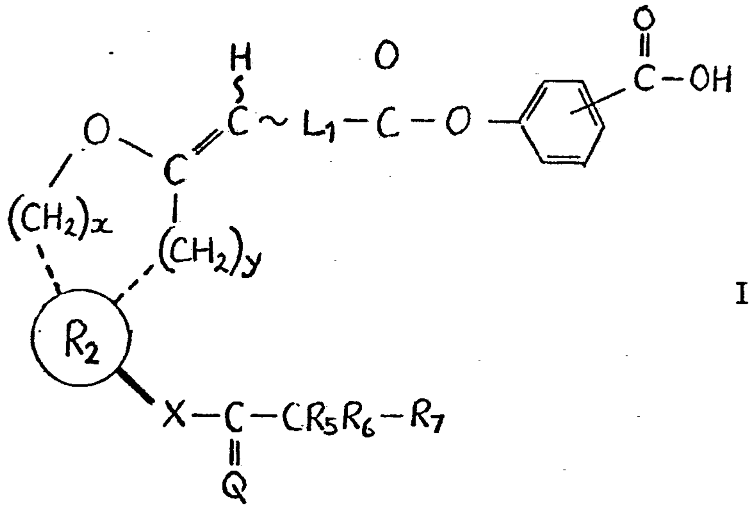

II

The compounds of this invention are acylphenyl esters of prostaglandins which include 5-oxo-$PGF_{1\alpha}$ and its analogues, 6-oxo-$PGF_{1\alpha}$ and its analogues, and 6-oxo-9-deoxy-9α-hydroxymethyl-$PGF_1$. In the esterifying group, the acyl substituent on the phenyl group may be, for example, acetyl, propionyl, butyryl, isobutyryl, valeryl or isovaleryl in any of the 2, 3 and 4-positions. Alternatively, the esterifying group may be 4-pivalylphenyl. This group is preferably 4-acetylphenyl.

The esters of this invention are useful for the same purposes as the parent prostaglandins and their salts. They may therefore be used by oral, sublingual, buccal, rectal, intravaginal, intrauterine or topical administration, or by inhalation.

The novel acyl-substituted phenyl esters have advantages over the corresponding known prostaglandin compounds in that they can be more stable. They are often obtained in crystalline form, allowing ease of handling, and can show greater efficacy than the corresponding free acids or salts. The crystalline esters also provide a means of purifying the parent prostaglandin compound by recrystallisation.

Processes for the preparation of the compounds of the invention will now be described. By way of example, the compounds may be prepared starting from the corresponding prostaglandin acid or salt of the formula

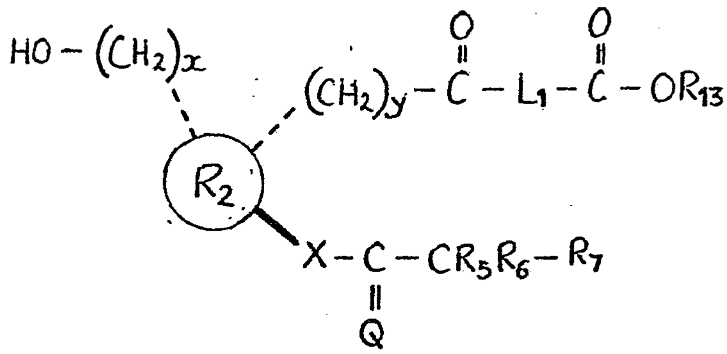

III

wherein $x$, $y$, $L_1$, Q, $R_1$, $R_5$, $R_6$, $R_7$ and X are as defined above and $R_{13}$ is hydrogen, sodium, potassium or lithium.

Compounds of formula III in which y is one are disclosed in, for example, Belgian Patent Specification No. 851,122. Examples of such compounds are 6-oxo-$PGF_{1\alpha}$ and its analogues, including the following:

6-oxo-trans-$\Delta^2$-$PGF_1$
6-oxo-2,2-difluoro-$PGF_{1\alpha}$
6-oxo-11β-$PGF_{1\alpha}$
6-oxo-11-deoxy-$PGF_{1\alpha}$
6-oxo-13,14-dihydro-$PGF_{1\alpha}$
6-oxo-13,14-didehydro-$PGF_{1\alpha}$
6-oxo-15-deoxy-$PGF_{1\alpha}$
6,15-dioxo-$PGF_{1\alpha}$
6-oxo-(15S)-15-methyl-$PGF_{1\alpha}$
6-oxo-(15R)-15-methyl-$PGF_{1\alpha}$
6-oxo-13,14-didehydro-(15S)-$PGF_{1\alpha}$
6-oxo-13,14-didehydro-(15R)-$PGF_{1\alpha}$
6-oxo-16,16-dimethyl-$PGF_{1\alpha}$
6-oxo-16,16-difluoro-$PGF_{1\alpha}$

6-oxo-17,18-didehydro-$PGF_{1\alpha}$

6-oxo-17-phenyl-18,19,20-trinor-$PGF_{1\alpha}$

6-oxo-16-phenoxy-17,18,19,20-tetranor-$PGF_{1\alpha}$

6-oxo-9-deoxy-9α-hydroxymethyl-$PGF_1$

6-oxo-9-deoxy-9α-hydroxymethyl-11β-$PGF_1$

6-oxo-9,11-dideoxy-9α-hydroxymethyl-$PGF_1$

6-oxo-9-deoxy-9α-hydroxymethyl-13,14-dihydro-$PGF_1$

6-oxo-9-deoxy-9α-hydroxymethyl-13,14-didehydro-$PGF_1$

6-oxo-9-deoxy-9α-hydroxymethyl-17,18-didehydro-$PGF_1$ and

6-oxo-9-deoxy-9α-hydroxymethyl-17-phenyl-18,19,20-trinor-$PGF_1$

Compounds of formula III in which x is zero and y is 2 are disclosed in, for example, U.S. Patent Specifications Nos. 4,109,082 and 4,110,532. Examples of such compounds are 5-oxo-$PGF_{1\alpha}$ and its analogues, including the following:

5-oxo-11-deoxy-$PGF_{1\alpha}$

5-oxo-13,14-dihydro-$PGF_{1\alpha}$

5-oxo-13,14-didehydro-$PGF_{1\alpha}$

5-oxo-(15S)-15-methyl-$PGF_1$ and

5-oxo-17-phenyl-18,19,20-trinor-$PGF_{1\alpha}$

In the first step of one process for preparing compounds of the invention, a mixed anyhdride of a formula III compound is prepared by reacting that compound at -40 to +60°C, preferably -10 to +10°C, with isobutyl chloroformate. The latter is preferably used in excess, for example from 1.2 to 4 molar equivalents per mole of the prostaglandin compound. At the given temperature, the reaction rate is reasonably fast, and yet side-reactions are minimised.

The reaction is preferably conducted in a solvent. For this purpose, acetone is preferred, although other relatively non-polar solvents, such as acetonitrile, dichloromethane or chloroform, may be used. The reaction is conducted in the presence of a tertiary amine such as triethylamine, and the amine hydrochloride which is

formed usually crystallises out, but need not be removed for the second step of the process.

In the second step, $R_{13}$ having been replaced by an isobutyl group to form a mixed anhydride, this is reacted with an acylphenol (or hydroxyphenyl alkyl ketone) of the formula

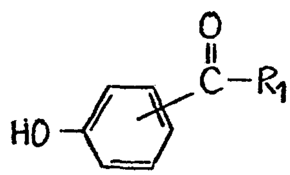

wherein $R_1$ is as defined above.

The anhydride is usually not isolated but is reacted directly in solution with the acylphenol, preferably in the presence of a tertiary amine such as pyridine or triethylamine. Suitable acylphenols are known in the art or readily available by methods known in the art.

The acylphenol is preferably used in an amount equivalent to, or in excess of, the anhydride, in order to insure that all of the mixed anhydride is converted to the desired ester. Excess acylphenol may be separated from the product by methods described herein or known in the art, for example by crystallization. The tertiary amine is not only a basic catalyst for the esterification but also a convenient solvent. Other examples of tertiary amines useful for this process include N-methylmorpholine, triethylamine, diisopropylethylamine and dimethylaniline. The use of 2-methylpyridine or quinoline results in a slow reaction. A highly hindered amine such as 2,6-dimethyllutidine is not useful because of the slowness of the reaction.

The reaction with the anhydride proceeds smoothly at room temperature (about 20 to 30°C) and can be followed in conventional manner with thin layer chromatography (TLC). It is usually found to be complete within 1-4 hours.

The reaction mixture is worked up to yield the ester following methods known in the art, and the product is purified, for example by silica gel chromatography.

Solid esters may be converted to a free-flowing crystalline form on crystallization from a variety of solvents, including ethyl acetate, tetrahydrofuran, methanol and diethyl ether, by cooling or evaporating a saturated solution of the ester in the solvent or by adding a miscible non-solvent such as hexane or water. The crystals are then collected by conventional techniques, e.g. filtration or centrifugation, washed with a small amount of solvent, and dried under reduced pressure. They may also be dried in a current of warm nitrogen or argon, or by warming to about 75°C. Although the crystals are normally pure enough for many applications, they may be recrystallized by the same general techniques to achieve improved purity after each re-crystallization.

In an alternative two-step process, a compound of formula III is first reacted with a 2-halo-1-methylpyridinium iodide, thereby replacing $R_{13}$ by a 1-methyl-2-pyridinium iodide group and forming a pyridinium ester intermediate. This method has been applied to other carboxylic esters by T. Mukaiyama et al., Chemistry Letters 1975, pp. 1045-1048. 2-Halo-1-methylpyridinium iodides are known or readily available; for example, the 2-bromo compound is described by G.B. Barlin et al., J. Chem. Soc., Perkins Trans., 2 (1974) 790.

The 2-halo-1-methylpyridinium iodide is preferably used in an equivalent amount or in excess. The reaction is preferably conducted in the presence of a tertiary amine which is used in an equivalent amount or in excess with respect to an alkali metal salt of formula III, or in double that amount for a free acid of the prostacyclin-type compound. Useful tertiary amines are those named above, including triethylamine.

The reaction with the 2-halo-1-methylpyridinium iodide proceeds smoothly at room temperature (about 20 to 30°C) and can be carried out at temperatures up to

about 120°C. The reaction is conveniently run in a solvent such as toluene, methylene chloride, dimethoxyethane, pyridine or dimethylformamide.

The resulting pyridinium ester is usually not isolated but is reacted directly in solution, in the second step of the process, with an acylphenol as described above. The reaction is preferably conducted in the presence of a tertiary amine such as was used in the first step.

The reaction mixture may be worked up by conventional methods, including two-phase extraction, silica gel column chromatography and crystallization.

Another process for preparing a compound of the invention comprises hydrolysing a compound of formula II. The reaction may be conducted in the presence of a mild acid such as aqueous potassium hydrogen sulphate.

The following Example illustrates the invention.

Example

6-Oxo-$PGF_{1\alpha}$,4-Acetylphenyl Ester

A mixture of $PGI_2$ sodium salt (0.561 g.) in triethylamine (0.200 g.) in 15 ml. of dimethylformamide is treated at about 25°C. with 0.45 g. of solid 2-bromo-1-methylpyridinium iodide and stirred for 1.2 hr. A solution of 4-hydroxyacetophenone (0.204 g.) in 1 ml. of dimethylformamide is then added, followed by about 0.20 ml. of triethylamine. After standing overnight, one major spot is observed on thin layer chromatography (TLC).

The mixture is treated with 5 ml. of 2 N aqueous potassium hydrogen sulphate and, within 15 minutes, the mixture contains a more polar material as shown by TLC. After further stirring for 1.5 hr, the mixture is poured into 70 ml. of ice-water and extracted with diethyl ether. The organic phase is washed with an aqueous saturated sodium chloride solution, dried by contact with an anhydrous agent, filtered to remove solids and concentrated under reduced pressure to an oil, 0.680 g. The oil is chromatographed, eluting

with ethyl acetate, to obtain the title compound, 0.175 g.

The product has $R_f$ (the ratio in TLC, on silica gel in ethyl acetate, between the movement of a spot of the sample and that of the solvent front) = 0.25, and NMR peaks at 8.05, 7.29, 5.72-5.40, 4.83-3.50, 3.50-3.02, 3.02-1.08, 2.58 and 0.89 δ. The NMR spectrum was recorded on a Varian (registered Trade Mark) A-60, A-60D, T-60 or XL-100 spectrophotometer in deuterochloroform solution with tetramethylsilane as an internal standard.

CLAIMS

1. A compound of the formula

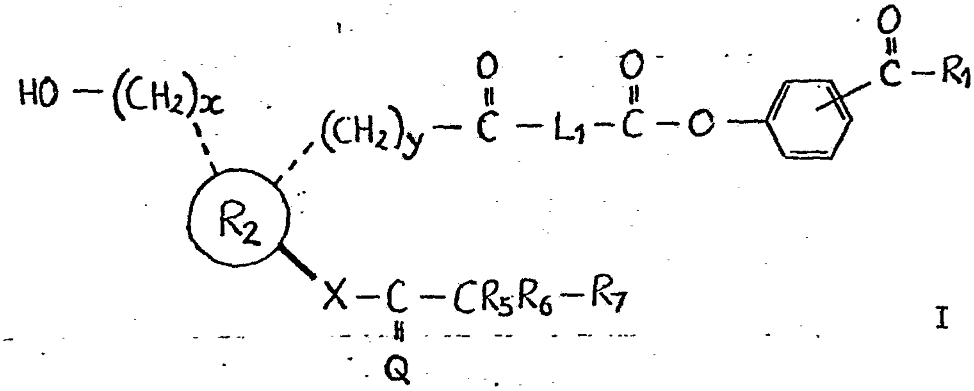

I

wherein x is zero or one, y is one or 2, and x plus y is one or 2;

$L_1$ is $-(CH_2)_n-$, $-(CH_2)_p-CF_2-$ or $-(CH_2)_v-CH=CH-$ and n is an integer of from one to 5, p is 2, 3 or 4 and v is one, 2 or 3;

Q is oxo, α-H:β-H, α-OH:β-$R_4$ or α-$R_4$:β-OH and $R_4$ is hydrogen or $C_{1-4}$ alkyl;

$R_1$ is $C_{1-4}$ alkyl, provided that $-COR_1$ is in the 4-position on the benzene ring when $R_1$ is tert-butyl;

($R_2$) is

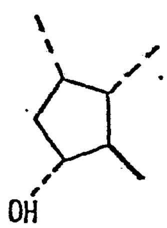

,

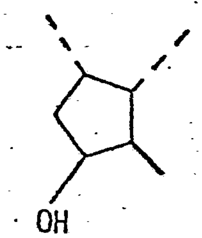

,

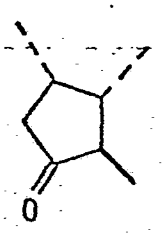

,

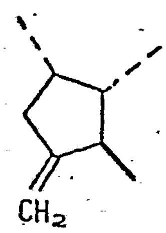

,

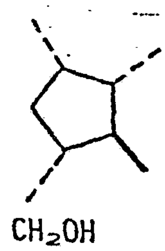

,

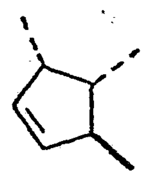

or

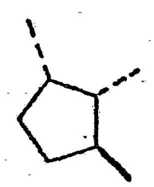

$R_7$ is $C_{2-10}$ alkyl (with a straight chain no longer than 6 carbon atoms) or optionally ring-substituted phenoxy, phenyl or phenyl($C_{1-9}$ alkyl) with no more than 6 carbon atoms in the chain between $CR_5R_6$ and the benzene ring, in which there are up to 3 ring substituents independently selected from fluorine, chlorine, trifluoromethyl, $C_{1-4}$ alkyl and $C_{1-4}$ alkoxy, provided that no more than two substituents are other than alkyl;

$R_5$ and $R_6$ are independently selected from hydrogen, fluorine and $C_{1-4}$ alkyl, with the provisos that $CR_5R_6$ is not CFAlkyl and that neither $R_5$ nor $R_6$ is fluorine when $R_7$ is optionally ring-substituted phenoxy; and

X is <u>trans</u>-CH=CH-, <u>cis</u>-CH=CH-, -C≡C- or $-CH_2CH_2-$.

2. 6-Oxo-$PGF_{1\alpha}$, 4-acetylphenyl ester.

3. 6-Oxo-trans-$\Delta^2$-$PGF_{1\alpha}$, 4-acetylphenyl ester.

4. 6-Oxo-2,2-difluoro-$PGF_{1\alpha}$, 4-acetylphenyl ester.

5. 6-Oxo-11-deoxy-$PGF_{1\alpha}$, 4-acetylphenyl ester.

6. The 4-acetylphenyl ester of 6-oxo-13,14-dihydro-$PGF_{1\alpha}$ or 6-oxo-13,14-didehydro-$PGF_{1\alpha}$, in either (15S) or (15R) configuration.

7. The 4-acetylphenyl ester of any of
   6-oxo-15-deoxy-$PGF_{1\alpha}$;
   6-oxo-(15S)-15-methyl-$PGF_{1\alpha}$;
   6-oxo-(15R)-15-methyl-$PGF_{1\alpha}$;
   6-oxo-16,16-dimethyl-$PGF_{1\alpha}$;
   6-oxo-16,16-difluoro-$PGF_{1\alpha}$;
   6-oxo-17,18-didehydro-$PGF_{1\alpha}$;
   6-oxo-17-phenyl-18,19,20-trinor-$PGF_{1\alpha}$; and
   6-oxo-16-phenoxy-17,18,19,20-tetranor-$PGF_{1\alpha}$.

8. 5-Oxo-$PGF_{1\alpha}$, 4-acetylphenyl ester.

9. 6-Oxo-9-deoxy-9α-hydroxymethyl-$PGF_1$, 4-acetylphenyl ester.